(19) Europäisches Patentamt
European Patent Office
Office européen des brevets

(11) Publication number: 0 172 463 B1

(12) # EUROPEAN PATENT SPECIFICATION

(45) Date of publication of the patent specification: 09.05.90

(51) Int. Cl.5: C07D 277/40, C07D 277/46

(21) Application number: 85109618.0

(22) Date of filing: 31.07.85

(54) **A process for producing thiazole glutaconic acid derivatives.**

(30) Priority: 24.08.84 JP 176954/84

(43) Date of publication of application:
26.02.86 Bulletin 86/9

(45) Publication of the grant of the patent:
09.05.90 Bulletin 90/19

(84) Designated Contracting States:
DE FR IT NL

(56) References cited:
EP-A- 0 049 449
EP-A- 0 081 674
EP-A- 0 136 721
EP-A- 0 168 025

(73) Proprietor: Shionogi & Co., Ltd., 1-8, Doshomachi 3-chome, Chuo-ku, Osaka 541/Japan(JP)

(72) Inventor: Onoue, Hiroshi, 3-7-4, Takatsukadai Kawai-cho, Kitakatsuragi-gun Nara(JP)
Inventor: Takahashi, Hiromi, 5-6-37-204, Hiyodoridai Kita-ku, Kobe-shi Hyogo(JP)

(74) Representative: Bruin, Cornelis Willem et al, Octrooibureau Arnold & Siedsma Isartorplatz 5, D-8000 München 2(DE)

EP 0 172 463 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

This invention relates to the synthesis of thiazoleglutaconic acid derivatives which are useful as starting materials for the side chain of antibacterially active penicillin and cephalosporin derivatives.

Thiazoleglutaconic acid derivatives of this type, as well as their methods of preparation, have been described earlier in EP-A 0 081 674, EP-A 0 136 721, EP-A 0 168 025.

The invention provides a new production process which makes use of a Michael-type addition.

According to the invention, a thiazoleacetic acid derivative (I) is reacted with an alkoxyacrylic acid derivative (II) in the presence of a base to obtain a thiazoleglutaconic acid derivative (III):

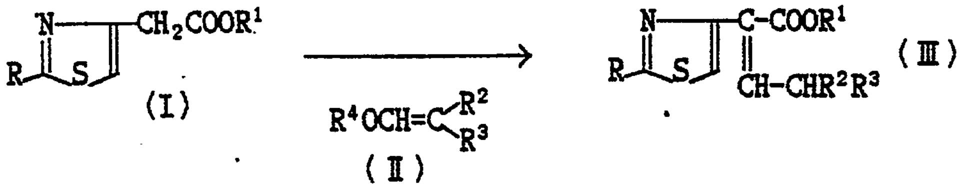

(I) (II) (III)

The resulting thiazoleglutaconic acid derivative (III) may thereupon be deprotected (e.g. by hydrolysis, in some cases accompanied by decarboxylation) to form a thiazoleglutaconic acid derivative (IV) which is useful for the preparation of antibacterially active penicillin and cephalosporin derivatives.

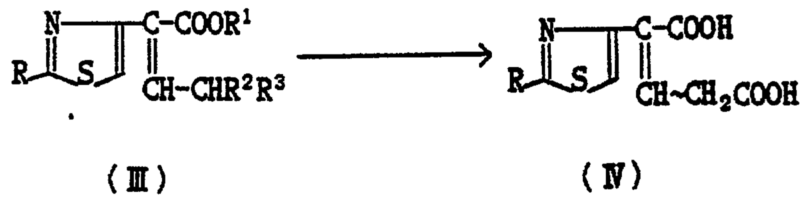

(III) (IV)

In these formulae, the meaning of the symbols is as follows:

R is an amino group which may be protected by (C1–C5)-alkanoyl (e.g. formyl, acetyl, isobutyryl), halogeno-(C1–C5)-alkanoyl (e.g. chloroacetyl), (C2–C6)-alkoxycarbonyl (e.g. methoxycarbonyl, t-butoxycarbonyl, trichloroethoxycarbonyl, iodoethoxycarbonyl), (C8–C15)-aralkoxycarbonyl (e.g. benzyloxycarbonyl, dimethylbenzyloxycarbonyl, nitrobenzyloxycarbonyl), (C1–C15)-alkylsulfonyl or (C1–C15)-arylsulfonyl (e.g. methanesulfonyl, ethanesulfonyl, benzenesulfonyl, toluenesulfonyl, bromobenzenesulfonyl), or (C19–C20)-triarylmethyl (e.g. triphenylmethyl), a (C3–C10)-enamine forming group, tri-(C1–C5)-alkylsilyl (e.g. tertbutyl-dimethylsilyl, trimethylsilyl), a (C2–C10)-Schiff base-forming group (e.g. dimethylaminomethylidene, benzylidene).

$R^1$ is an ester forming group selected from (C1–C8)-alkyl (e.g. methyl, ethyl, propyl, isopropyl, butyl, t-butyl), (C1–C5)-alkyl substituted by halogen or (C1–C5)-alkoxy or (C1–C8)-alkanesulfonyl (e.g. chloromethyl, trichloromethyl, methoxyethyl, methanesulfonylethl); (C2–C5)-alkenyl (e.g. vinyl, propenyl, prenyl); (C7–C15)-aralkyl (e.g. benzyl, methylbenzyl, nitrobenzyl, methoxybenzyl, diphenylmethyl), or other conventional carboxy-protecting groups;

$R^2$ and $R^3$ each are hydrogen, cyano, or an esterified carboxyl group which carries one of the ester-forming groups defined for $R^1$; and

$R^4$ is (C1–C5)-alkyl (e.g. methyl, ethyl, propyl, butyl, isobutyl, especially methyl and ethyl) or (C7–C15)-aralkyl (e.g. benzyl, methylbenzyl).

The Michael-type addition of this invention has in reality a complicated reaction sequence, viz. first a reaction of the thiazole acetic acid derivate (I) with the base to form a carbanion-containing compound (Ia), next an addition of this compound (Ia) to the alkoxyacrylic acid derivative (II) to form a thiazolealkoxyglutaric acid derivative (Ib) and thereupon the elimination of an alcohol $R^4OH$ from the latter to give the desired thiazoleglutaconic acid derivative (III). Compare the following reaction diagram:

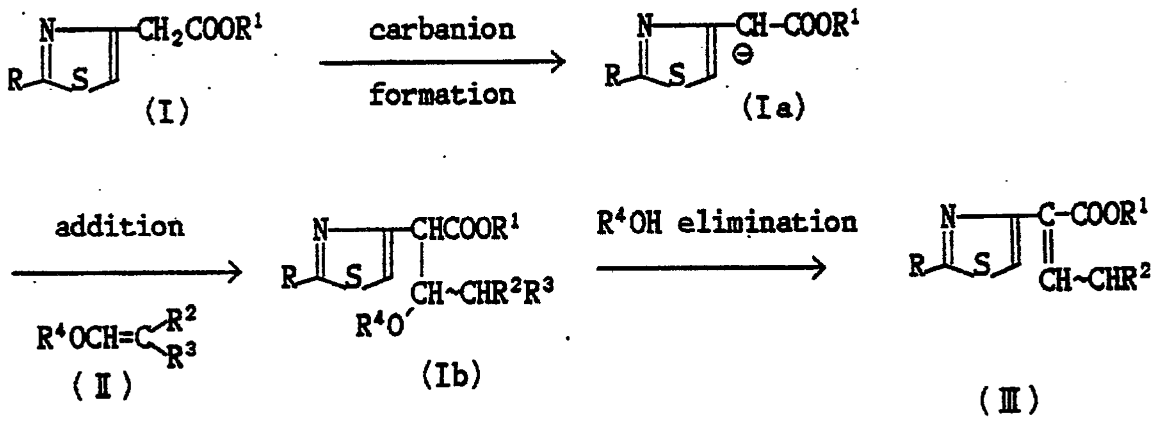


(I) (Ia) (II) (Ib) (III)

Each of the reactions of carbanion formation, addition, and alcohol-elimination may proceed in the presence of a base under anhydrous conditions. This means that an isolation of the intermediates (Ia) and (Ib) is not always necessary and that the whole process starting from the thiazoleacetic acid derivative (I) and ending with the thiazoleglutaconic acid derivative (III) may be effected in a single step, especially when $R^2$ in the alkoxyacrylic acid derivative (II) is hydrogen. In other cases, the process may be effected in two steps, with isolation of the intermediate compound (Ib).

The base to be used in the reactions can be a metal compound, e.g. an alkalimetalhydride or alkoxide.

The reactions of carbanion-formation and addition are carried out under anhydrous conditions. They proceed well by contacting the alkoxyacrylic acid derivative (II) (preferably in a proportion of 1 to 5 equivalents, especially 1 to 3 equivalents) with the thiazoleacetic acid derivative (I) and the base. The resulting addition product (Ib) may sometimes be isolated from the reaction mixture in a conventional way.

The elimination of an alcohol $R^4OH$ is effected by the action of a base on the addition product (Ib). This reaction may proceed under anhydrous conditions, just like the preceding reactions, but it may also proceed in a polar solvent (e.g. water).

The thus produced thiazoleglutaconic acid derivative (III) is usually a mixture of geometrically isomers in relation to the double bond. The over-all yield, calculated from the thiazoleacetic acid derivative (I) may amount to over 90%.

Deprotection of this derivative (III) (e.g. by hydrolysis) gives a thiazoleglutaconic acid derivative (IV). When $R^2$ and $R^3$ in the derivative (III) are both esterified carboxyl groups, a decarboxylation will occur simultaneously with the deprotection, thus resulting into the same thiazoleglutaconic acid derivative (IV).

The above reactions proceed well in a solvent. Such solvent can be an ether (e.g. dimethoxyethane, tetrahydrofuran, dioxane), a nitrile (e.g. acetonitrile, propionitrile, benzonitrile), a sulfoxide (e.g. dimethylsulfoxide), an amide (e.g. N,N-dimethylformamide, N,N-dimethylacetamide, hexamethylphosphorotriamide), an ester (e.g. methyl formate, ethyl acetate), or a mixture thereof. Further, the alkoxy acrylic acid derivative (II) may serve as a solvent. Each reaction is usually carried out at –30° to 100°C (especially –20° to 70°C) and often during a period from 5 minutes to 10 hours.

The starting materials, i.e. the thiazoleacetic acid derivative (I) and the alkoxyacrylic acid derivative (II) are known compounds or easily available from known compounds.

The invention is illustrated by the following examples. In the examples, "parts" are parts by weight and "equivalents" are molar equivalents. Further, the following abbreviations are used:

BOC = t-butoxycarbonyl; Bzl = benzyl; Cbz = benzyloxycarbonyl; DBU = diazabicycloundecene; DMA = dimethylacetamide; DMF = dimethylformamide; Et = ethyl; Me = methyl; nd = not determined; Ph = phenyl; rt = room temperature; and THF = tetrahydrofuran.

Example 1A (Addition)

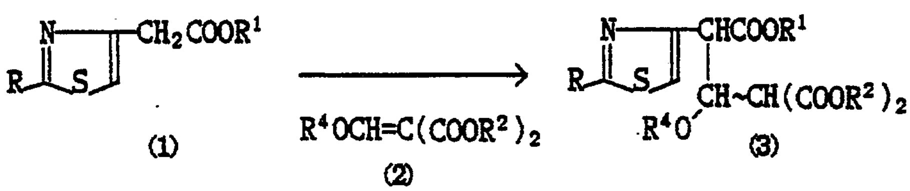


(1) (2) (3)

To a suspension of sodium hydride in tetrahydrofuran is added a solution of a thiazoleacetic acid ester (1) and a 2-(alkoxymethylidene)malonic acid ester (2) in tetrahydrofuran. After stirring at a given temperature for a given time, the mixture is diluted with ethyl acetate, neutralized with acetic acid, washed with water, dried, and concentrated in vacuo. The residue gives, if required after purification by chromatography, a thiazolealkoxyglutaric acid ester (3). Geometric isomers can be isolated.

The reaction conditions are listed in Table 1 and the physical constants of the products are listed in Table 5.

Example 1B (Elimination)

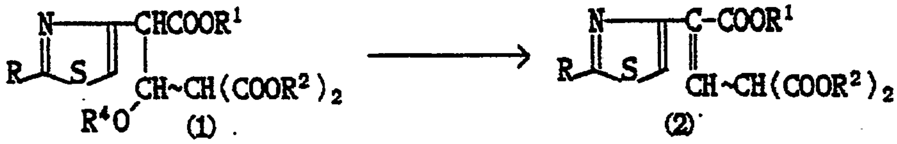
(1) (2)

To a solution of a thiazolealkoxyglutaric acid derivative (1) in a solvent is added a given base. The mixture is stirred at a given temperature for a given time. Thereupon, the mixture is neutralized, diluted with water and ethyl acetate, washed with water, dried, and concentrated in vacuo. The residue gives, if required after purification by silica gel chromatography, a thiazolecarboxyglutaconic acid ester derivative (2). Geometric isomers can be isolated.

The reaction conditions are listed in Table 2 and the physical constants of the products are listed in Table 6.

Example 2 (Hydrolysis and decarboxylation)

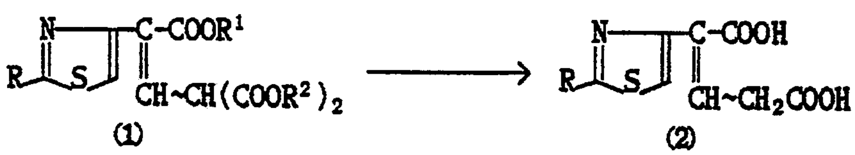
(1) (2)

To a solution of a thiazolecarboxyglutaconic acid ester (1) in a given solvent is added a given base. After stirring at a given temperature for a given time, the mixture is diluted with water and ethyl acetate. The aqueous layer is separated acidified with hydrochloric acid and extracted with ethyl acetate. The extract is washed with water, dried, and concentrated in vacuo. The residue is a thiazoleglutaconic acid derivative (2).

The reaction conditions are listed in Table 3 and the physical constants of the products are listed in Table 7.

Example 3 (Addition and elimination)

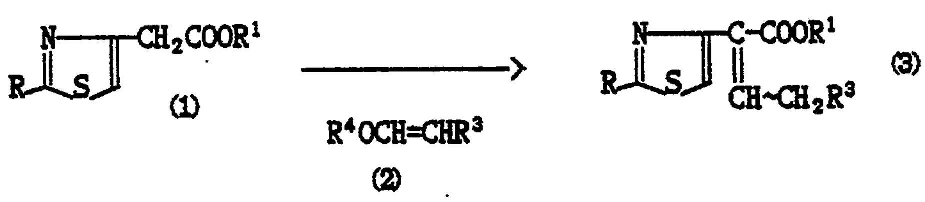

(1) (3)

A solution of a base, an alkoxyacrylic acid derivative (2) and a thiazoleacetic acid derivative (1) in N,N-dimethylformamide is kept at a given temperature for a given time. The reaction mixture is poured into a mixture of 5% hydrochloric acid and ethyl acetate. The organic layer is separated, washed with water, dried, and concentrated in vacuo to give a thiazoleglutaconic acid ester derivative (3), if required after purification by chromatography.

When the base is a hydride, a trace amount of alcohol can be added as a reaction initiator.

The reaction conditions are listed in Table 4 (Part 1) and the physical constants of the products are listed in Table 8.

Example 4 (Addition and elimination)

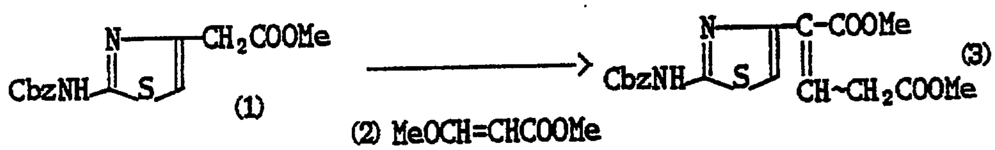

(1) (3)

To a solution of 2-(2-benzyloxycarbonylamino-4-thiazolyl)-acetic acid methyl ester (1) in a given solvent are added 3-methoxyacrylic acid methyl ester (2) and sodium methoxide. After reacting at 0 to 15°C for 15 minutes, the mixture is analyzed by high performance liquid chromatography to determine the yield of 2-(2-benzyloxycarbonylamino-4-thiazolyl)glutaconic acid dimethyl ester (3).

The reactions and yields are listed in Table 4 (Part 2).

Preparation 1

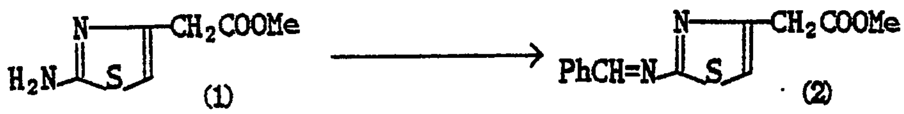
(1) (2)

To a solution of an amine (1) in toluene (4.3 parts) is added benzaldehyde (5 equivalents). After refluxing under azeotropic conditions for 2 hours, the mixture is concentrated to remove the solvent and benzaldehyde. The residue is purified by silica gel chromatography (hexane:acetone = 3:2) to give a Schiff base (2). Mp. 71–72°C (hexane – ether). Yield: 62.8%.

IR(Nujol)ν: 1730, 1605, 1585, 1160 $cm^{-1}$.

NMR ($CDCl_3$)δ: 3,71(s, 3H), 3.80(s, 2H), 7.07(s, 1H), 7.47(m, 3H), 7.93(m, 2H), 8.98(s, 1H) ppm.

Preparation 2

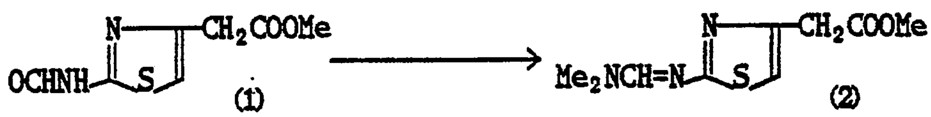
(1) (2)

Phosphorus oxychloride (5 equivalents) is added to N,N-dimethylformamide (1.8 parts) at 6 to 11°C for 18 minutes and the mixture is diluted with chloroform (4.5 parts). The solution is added to a suspension of an N-formyl compound (1) in chloroform (10 parts) at 7°C during 15 minutes. The mixture is stirred at room temperature for 140 minutes. The reaction mixture is cooled to 5°C, neutralized with aqueous 3N-sodium hydroxide to pH 7, and extracted with chloroform. The extract is washed with water, dried, and concentrated in vacuo. The residue is purified by silica gel chromatography (ethyl acetate:acetonitrile = 8:2) to give a carbodiimide (2). Yield: 58.2%.

IR($CHCl_3$)ν: 1730, 1630, 1100 $cm^{-1}$.

NMR($CDCl_3$)δ: 3.03, 3.06(2×s, 6H), 3.70(s, 5H), 6.60(s, 1H), 8.17(s, 1H) ppm.

Preparation 3

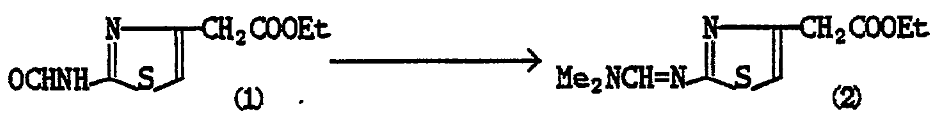
(1) (2)

An N-formyl compound (1) is treated in the same manner as in Preparation 2 giving a carbodiimide (2). Yield: 87.7%.

IR($CHCl_3$)ν: 1730, 1620, 1103 $cm^{-1}$.

NMR($CHCl_3$)δ: 1.26(t, J=7Hz, 3H), 3.04(s, 6H), 3.63(s, 2H), 4.15(q, J=7Hz, 2H), 6.60(s, 1H), 8.14(s, 1H) ppm.

Preparation 4

$$\underset{(1)}{MeOCH{=}CHCOOMe} \xrightarrow[NaOMe]{PhCH_2OH} \underset{(2)}{PhCH_2OCH{=}CHCOOCH_2Ph}$$

A mixture of 3-methoxyacrylic acid methyl ester (1), benyl alcohol (1.2 equivalents), a solution of sodium methoxide in methanol (0.05 equivalents), and toluene (3.7 parts) is heated for 3 hours to remove methanol by azeotropic distillation. The mixture is concentrated in vacuo to remove the solvent, distilled in vacuo to remove by-products (94 to 112°C at 3 mmHg), and colled. The residue is chromatographed over silica gel (benzene:ethyl acetate = 95:5) to give the 3-benzyloxyacrylic acid benzyl ester (2). Yield: 45%.

NMR($CDCl_3$)δ: 4.74(s, 2H), 5.11(s, 2H), 5.32(d, J=13H, 1H), 7.29(m, 10H, 7.69(d, J=13Hz, 1H) ppm.

Table 1 (Addition)

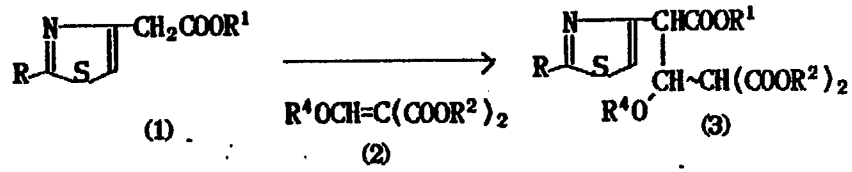


(1) (3)

| No. | R | $R^1$ | $R^2$ | $R^4$ | NaH (equiv.) | THF (part) | reagent (equiv.) | THF (part) | temp (°C) | time (min) | yield (%) | stereoisomers A (isolated) B |
|---|---|---|---|---|---|---|---|---|---|---|---|---|
| 1 | BOCNH | Me | Et | Et | 2.0 | 5.7 | 1.2 | 5.7 | 20 | 210 | 52.7 | 21.9 30.8 |
| 2 | CbzNH | Me | Me | Me | 2.3 | 4.8 | 1.6 | 9.7 | 10<br>rt | 10<br>180 | 84 | mixture |
| 3 | CbzNH | Me | Et | Et | 2.2 | 5.8 | 1.4 | 5.8 | rt<br>40<br>rt | 15<br>30<br>150 | 31.7 | 11.5 20.2 |
| 4 | OCHNH | Et | Et | Et | 2.0 | 20.5 | 1.2 | - | rt | 120 | 60 | 4:5 mixture |
| 5 | PhCH=N | Me | Et | Et | 2.0 | 8.9 | 2.0 | 7.1 | 8<br>rt<br>60 | 10<br>210<br>60 | 40 | |
| 6 | $Me_2NCH=N$ | Me | Me | Me | 3.0 | 8.8 | 1.5 | 6.1 | 3<br>rt<br>60 | 7<br>60<br>45 | >85 | continued in Table 2, No. 7. |
| 7 | $Me_2NCH=N$ | Et | Et | Et | 3.0 | 8.6 | 1.5 | 86.3 | rt<br>60 | 120<br>40 | >60 | continued in Table 2, No. 8. |

Table 2 (Elimination)

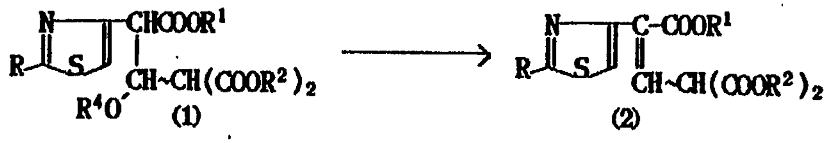

(1) (2)

| No. | R | $R^1$ | $R^2$ | $R^4$ | solvent (part) | base (equiv.) | temp (°C) | time (min) | yield (%) | cis : trans |
|---|---|---|---|---|---|---|---|---|---|---|
| 1 | BOCNH | Me | Et | Et | MeOH 3.6 | 2N-KOH 4.0 | 0 | 15 | 86 | 3 : 4 (from isomer A) |
| 2 | BOCNH | Me | Et | Et | MeOH 3.6 | 2N-KOH 4.0 | 0 | 15 | – | 3 : 4 (from isomer B) |
| 3 | CbzNH | Me | Me | Me | $CH_2Cl_2$ 19.6 | 2N-KOH 10.0 | rt | 300 | – | continued in Table 3, No. 2 |
| 4 | CbzNH | Me | Et | Et | MeOH 3.1 | 2N-KOH 4.0 | 0 | 30 | 82 | 3 : 5 |
| 5 | OCHNH | Et | Et | Et | EtOH 3.5 | 2N-KOH 4.0 | rt | 60 | 79 | 5 : 6 |
| 6 | PhCH=N | Me | Et | Et | $CH_2Cl_2$ 25.5 | DBU 1.2 | rt | 120 | 38 | – |
| 7 | $Me_2NCH=N$ | Me | Me | Me | The same condition with Table 1, No. 6. | | | | 85 | (over-all yield from Table 1, No. 6) |
| 8 | $Me_2NCH=N$ | Et | Et | Et | The same condition with Table 1, No. 7. | | | | 60 | (over-all yield from Table 1, No. 7) |

T a b l e 3 (Hydrolysis + decarboxylation)

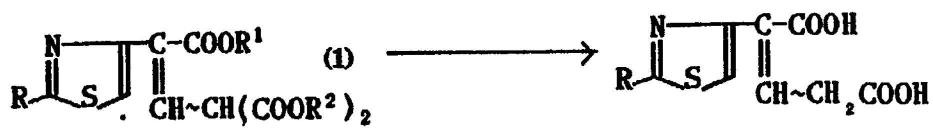

(1) (2)

| No. | R | $R^1$ | $R^2$ | Solvent (w/w) | Base (equiv.) | temp. (°C) | time (min) | yield (%) |
|---|---|---|---|---|---|---|---|---|
| 1 | BOCNH | Me | Et | MeOH (9.0) | 1N-NaOH (10.0) | rt | 360 | 34 |
| 2 | CbzNH | Me | Me | $CH_2Cl_2$ (19.6) | 2N-KOH (10.0) | rt | 300 | 57 (over-all from Table 2, No. 3) |
| 3 | CbzNH | Me | Et | MeOH (8.3) | 1N-NaOH (10.0) | rt | 300 | 26 |

Table 4 (Addition + Elimination) (Part 1)

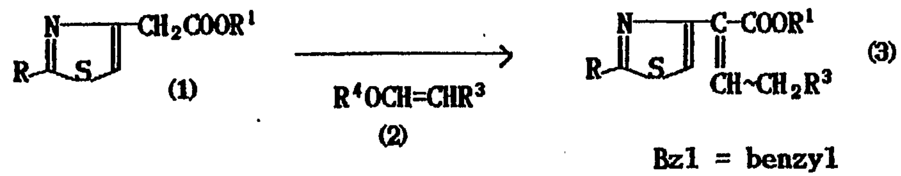


Bzl = benzyl

| No | R | $R^1$ | $R^3$ | $R^4$ | DMF (part) | base | equiv. | $R^4OH$ (equiv.) | reagent (equiv.) | temp (℃) | time (min) | yield (%) |
|---|---|---|---|---|---|---|---|---|---|---|---|---|
| 1 | BOCNH | Me | COOMe | Me | 6.3 | NaOMe | 3.1 | 0 | 2.4 | 0 | 40 | 60 |
| 2 | CbzNH | Me | COOMe | Me | 4.6 | NaOMe | 3.0 | 0 | 1.2 | 0 | 60 | 84 |
| 3 | CbzNH | Et | COOMe | Et | 11.8 | NaH | 2.4 | 0.2 | 1.4 | 15 | 30 | 68 |
| 4 | CbzNH | Bzl | COOBzl | Bzl | 4.1 | NaH | 3.0 | 0 | 1.6 | 10 | 60 | 46 |
| 5 | CbzNH | Bzl | COOBzl | Bzl | 4.1 | NaH | 3.0 | 0 | 1.5 | 10 | 70 | 36 |
| 6 | HCONH | Me | COOMe | Me | 18.9 | NaH | 2.5 | 2.0 | 2.0 | 0 | 30 | 78 |
| 7 | HCONH | Me | CN | Me | 18.9 | NaH | 2.5 | 1.0 | 2.0 | 0 | 40 | 42 |
| 8 | CbzNH | Me | CN | Me | 12.3 | NaH | 2.5 | 2.0 | 2.0 | 0 | 30 | 72 |
| 9 | $ClCH_2CONH$ | Me | COOMe | Me | 11.8 | NaH | 2.5 | 1.0 | 2.5 | -20 → 0 | 60 | 60 |

Table 4 (Addition + elimination) (Part 2)

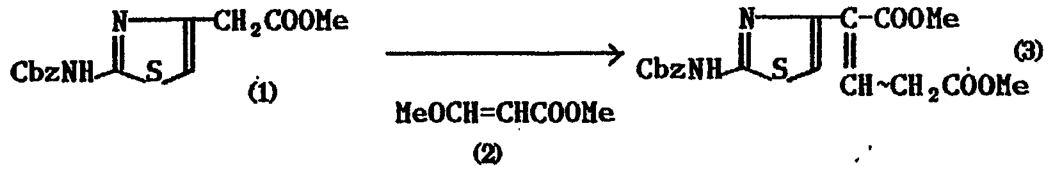


| No. | Solvent (Parts by volume) | NaOMe (equiv.) | Reagent (2) (equiv.) | Yield (3) (%) |
|---|---|---|---|---|
| 1 | MeCN (5) | 3 | 3 | 36 |
| 2 | THF (5) | 3 | 3 | 58 |
| 3 | DMSO (5) | 2.3 | 2.3 | 69 |
| 4 | DMF (5) | 3 | 3 | 78 |
| 5 | DMA (5) | 2.5 | 2 | 67 |
| 6 | 20% HCOOMe in DMA (5) | 2.5 | 2.0 | 62 |

| Effect of temperature on Reaction No. 4 | temperature (°C) | -20 | 4 | 18 | 33 | 42 | 58 |
|---|---|---|---|---|---|---|---|
| | yield of (3) (%) | 68.3 | 78.3 | 91.0 | 90.5 | 92.1 | 85.0 |

Table 5 (Part 1)

Physical constants of 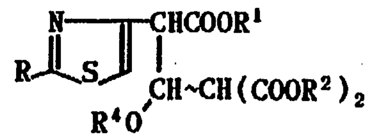

| No. | R | $R^1$ | $R^2$ | $R^4$ | IR($CHCl_3$) $\nu$ : $cm^{-1}$ | NMR($CDCl_3$) $\delta$ : ppm |
|---|---|---|---|---|---|---|
| 1A | BOCNH (Isomer A) | Me | Et | Et | nd. | 1.06(t, J=8Hz, 3H), 1.23(t, J=8Hz, 3H), 1.26(t, J=8 Hz, 3H), 1.52(s, 9H), 3.47(d, J=4Hz, 1H), 3.69(s, 3H), 3.70(m, 2H), 4.11(q, J=8Hz, 2H), 4.14(q, J=8Hz, 2H), 4.24(d, J=10Hz, 1H), 4.72(dd, $J_1$=4Hz, $J_2$=10Hz, 1H), 6.86(s, 1H), 8.23(s, 1H). |
| 1B | BOCNH (Isomer B) | Me | Et | Et | 3410, 1730. mp. 132~133°C | 0.97(t, J=8Hz, 3H), 1.21(t, J=8Hz, 3H), 1.24(t, J= 8Hz, 3H), 1.52(s, 9H), 3.47(m, 1H), 3.55(d, J=8Hz, 1H), 3.70(s, 3H), 4.12(m, 4H), 4.27(d, J=7Hz, 1H), 4.69(dd, $J_1$=7Hz, $J_2$=8Hz, 1H), 6.93(s, 1H), 8.38(s, 1H). |
| 2 | CbzNH | Me | Me | Me | 3395, 1734. | 3.31, 3.44(2×s, 3H), 3.57, 3.61, 3.63, 3.67, 3.70(5×s, 9H), 3.25~3.85(m, 1H), 4.31~4.68(m, 2H), 5.28(m, 2H), 6.92, 6.97(2×s, 1H), 7.37(s, 5H), 9.83(s, 1H). |

Table 5 (Part 2)

Physical constants of 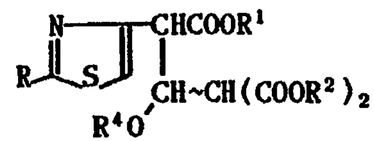

| No | R | $R^1$ | $R^2$ | $R^4$ | IR($CHCl_3$) ν :$cm^{-1}$ | NMR($CDCl_3$) δ :ppm |
|---|---|---|---|---|---|---|
| 3A | CbzNH (Isomer A) | Me | Et | Et | nd. | 1.04(t, J=8Hz, 3H), 1.18(t, J=8Hz, 3H), 1.20(t, J=8Hz, 3H), 3.48(d, J=4Hz, 1H), 3.64(s, 3H), 3.66(q, J=8Hz, 2H), 4.06(m, 2H), 4.08(q, J=8Hz, 2H), 4.35(d, J=9Hz, 1H), 4.68(dd, $J_1$=4Hz, $J_2$=9Hz, 1H), 5.26(s, 2H), 6.91(s, 1H), 7.34(s, 5H), 9.47(s, 1H). |
| 3B | CbzNH (Isomer B) | Me | Et | Et | nd. | 1.00(t, J=8Hz, 3H), 1.13(t, J=8Hz, 3H), 1.23(t, J=8Hz, 3H), 3.4~3.8(m, 3H), 3.57(s, 3H), 4.06(q, J=8Hz, 2H), 4.14(q, J=8Hz, 2H), 4.45(d, J=4Hz, 1H), 4.70(dd, $J_1$=4Hz, $J_2$=9Hz, 1H), 5.22, 5.36(ABq, J=12Hz, 2H), 7.00(s, 1H), 7.33(s, 5H), 10.17(s, 1H). |
| 4 | HCONH | Et | Et | Et | 3405, 3180, 1732, 1700. | 0.83~1.35(m, 12H), 3.1~3.8(m, 3H), 3.96~4.32(m, 7H), 4.52~4.74(m, 1H), 6.97, 7.05(2×s, 1H), 8.83(s, 1H), 11.77(s, 1H). |
| 5 | PhCH=N | Me | Et | Et | 1730, 1600, 1240. | 1.24(t, J=7Hz, 9H), 3.70(s, 3H), 4.14(m, 6H), 3.63, 4.12, (4.82(3×m, 3H), 7.22, 7.30(2×s, 1H), 7.33(m, 2H), 7.48(m, 3H). |

Table 6

Physical constants of 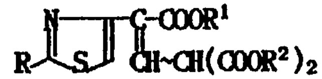

| No. | R | $R^1$ | $R^2$ | IR($CHCl_3$) $\nu$ : $cm^{-1}$ | NMR($CDCl_3$) $\delta$ : ppm |
|---|---|---|---|---|---|
| 1 | BOCNH | Me | Et | nd. | 1.22((t, J=8Hz, 3H), 1.24(t, J=8Hz, 3H), 1.50(s, 9H), 3.80, 3.84(2×s, 3H), 4.17(q, J=8Hz, 2H), 4.21(q, J=8Hz, 2H), 4.84, 5.13(2×d, J=9Hz, J=10Hz, 1H), 7.11(s, 1H), 7.15(m, 1H), 8.65(brs, 1H). |
| 2 | CbzNH | Me | Et | 3390, 1724. | 1.20, 1.23(2×t, J=8Hz, 6H), 3.73, 3.82(2×s, 3H), 4.16, 4.20(2×q, J=8Hz, 4H), 4.81, 4.97(2×d, J=9Hz, J=10Hz, 1H), 5.23(s, 2H), 5.1~5.4(m, 7H), 9.40(s, 1H). |
| 3 | HCONH | Et | Et | 3390, 3170, 1720. | 1.14~1.43(m, 9H), 4.06~4.44(m, 6H), 4.64~4.84(d, J=9Hz, J=10Hz, 1H), 6.97~7.25(m, 2H), 8.52, 8.53(2×s, 1H), 11.27(s, 1H). |
| 4 | PhCH=N | Me | Et | 1730, 1600, 1240. | 1.23(t, J=7Hz, 6H), 2.64(s, 3H), 4.19, 4.21(q, J=7Hz, 4H), 4.87, 5.12(2×d, J=10Hz, 1H), 7.23, 7.34(2×d, J=10Hz, 1H), 7.24, 7.31(2×s, 1H), 7.48, 7.74(2×m, 2H). |
| 5 | $Me_2NCH=N$ | Me | Me | 1755, 1715, 1610, 1165. (Nujol) mp. 88~89°C | 3.06(s, 3H), 3.12(s, 3H), 3.74(s, 6H), 3.81(s, 3H), 4.82, 5.27(2×d, J=10Hz, 1H), 6.94, 7.00(2×s, 1H), 7.15, 7.23(2×d, J=10Hz, 1H), 8.24, 8.41(2×s, 1H). |
| 6 | $Me_2NCH=N$ | Et | Et | 1725, 1615. | 1.24, 1.26(2×t, J=7Hz, 9H), 3.06, 3.10(2×s, 6H), 4.18(q, J=7Hz, 6H), 4.77, 5.17(2×d, J=10Hz, 1H), 7.03, 7.20(2×d, J=10Hz, 1H), 7.14(s, 1H), 8.23, 8.41(2×s, 1H). |

Table 7

Physical constants of 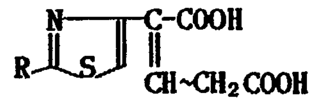

| No. | R | IR(Nujol) $\nu$ :$cm^{-1}$ | NMR $\delta$ :ppm |
|---|---|---|---|
| 1 | BOCNH (cis) | 3120, 1700, 1675. dp 153~154°C | 1.50(s, 9H), 3.45(d, J=7.5Hz, 2H), 7.00(t, J=7.5Hz, 1H), 7.13 (s, 1H). [$CD_3SOCD_3$] |
| 2 | BOCNH (trans) | 3150, 1700, 1630, 1600, dp 165~167°C | 1.49(s, 9H), 3.41(d, J=7.5Hz, 2H), 6.89(t, J=7.5Hz, 1H), 7.08 (s, 1H). [$CD_3SOCD_3$] |
| 3 | CbzNH | 3200, 1738, 1715, 1690, dp 169~172°C | 3.44, 3.50, (2×d, J=8Hz, 2H), 5.25(s, 2H), 7.07, 7.35(2×t, J=8Hz, 1H), 7.12(s, 1H), 7.38(brs, 5H). [$CDCl_3$+$CD_3OD$] |
| 4 | HCONH | 3400, 1718, 1690, 1630, 1550. dp 168°C | 3.45, 3.63(2×d, J=7.5Hz, 2H), 7.14, 7.32(2×t, J=7.5Hz, 1H), 7.23, 7.25(2×s, 1H), 8.51(s, 1H). [$CDCl_3$+$CD_3OD$] |

Table 8 (Part 1)

Physical constants of 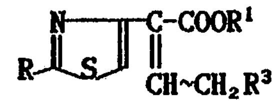

| No. | R | $R^1$ | $R^3$ | IR($CHCl_3$) $\nu$ : $cm^{-1}$ | NMR($CDCl_3$) $\delta$ : ppm |
|---|---|---|---|---|---|
| 1 | BOCNH (anti-isomer) | Me | COOMe | 3415, 1720 1541, 1155. | 1.52(s, 9H), 3.54(d, J=6.5Hz, 2H), 3.64(s, 3H), 3.76(s, 3H), 7.11(s, 1H), 7.18(t, 1H), 9.12(brs, 1H). |
| 2 | BOCNH (syn-isomer) | Me | COOMe | 3410, 1720, 1541, 1150. | 1.51(s, 9H), 3.54(d, J=6.5Hz, 2H), 3.69(s, 3H), 3.83(s, 3H), 7.03(s, 1H), 7.08(t, J=6.5Hz, 1H), 9.12(brs, 1H). |
| 3 | CbzNH | Me | COOMe | nd. | 3.41, 3.48(2×d, J=8Hz, 2H), 3.65, 3.73, 3.69, 3.83 (4×s, 6H), 5.24(s, 2H), 7.00~7.37(m, 7H). |
| 4 | CbzNH | Et | COOEt | 3395, 1720. | 1.19, 1.20, 1.22, 1.30(4×t, J=8Hz, 6H), 3.34, 3.42 (2×d, J=8Hz, 2H), 4.08, 4.12, 4.15, 4.24(4×q, J=8Hz, 4H), 5.21, 5.22, 5.24(3×s, 2H), 7.03, 7.13(2×t, J=8Hz, 1H), 7.03(s, 1H), 7.31(s, 5H), 10.15(brs, 1H). |
| 5 | CbzNH | $CH_2Ph$ | $COOCH_2Ph$ | 3400, 1725. | 3.31, 3.42(2×d, J=7Hz, 2H), 5.01, 5.03, 5.11, 5.17 (4×s, 6H), 6.96~7.30(m, 17H), 10.19(brs, 1H). |
| 6 | CbzNH | $CHPh_2$ | $COOCH_2Ph$ | 3490, 1725. | 3.34, 3.40(2×d, J=7Hz, 2H), 5.02, 5.05, 5.09, 5.17 (4×s, 4H), 6.8~7.4(m, 23H), 9.90(brs, 1H). |

Table 8 (Part 2)

Physical constants of 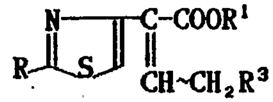

| No. | R | $R^1$ | $R^3$ | IR($CHCl_3$) ν : $cm^{-1}$ | NMR($CDCl_3$) δ :ppm |
|---|---|---|---|---|---|
| 7 | CbzNH | Me | CN | — | 3.73, 3.83(2×s, 3H), 5.23, 5.25(2×s, 3H), 6.78 (t, J=7Hz, 1H), 7.12, 7.23(2×s, 1H), 7.35(s, 5H), 9.82(brs, 1H). |
| 8 | HCONH | Me | CN | 3310, 2240, 1712, 1695sh (Nujol) | 3.80, 3.89(2×s, 3H), 6.85, 6.90(2×t, J=7Hz, 1H), 7.32, 7.51(2×s, 1H), 8.65(s, 1H), 11.15(brs, 1H). [$CD_3COCD_3$] |
| 9 | HCONH (anti-isomer) | Me | COOMe | — mp. 100°C | 3.46(t, J=7.5Hz, 2H), 3.66(s, 3H), 3.78(s, 3H), 7.05(s, 1H), 7.24(t, J=7.5Hz, 1H), 8.49(s, 1H). |
| 10 | HCONH (syn-isomer) | Me | COOMe | — | 3.56(d, J=7.0Hz, 2H), 3.73(s, 3H), 3.84(s, 3H), 7.02(t, J=7Hz, 1H), 7.12(s, 1H), 8.55(s, 1H). |
| 11 | $ClCH_2CONH$ (anti-isomer) | Me | COOMe | — | 3.50(d, J=6.5Hz, 2H), 3.68(s, 3H), 3.79(s, 3H), 4.25(s, 2H), 7.24(s, 1H), 7.24(t, 1H). |
| 12 | $ClCH_2CONH$ (syn-isomer) | Me | COOMe | — | 3.60(d, J=7Hz, 2H), 3.75(s, 3H), 3.87(s, 3H), 4.27(s, 2H), 7.18(s, 1H), 7.18(t, J=7Hz, 1H). |

## Claims

1. A process of preparing a thiazoleglutaconic acid derivative, which comprises reacting a thiazoleacetic acid derivative (I) with an alkoxyacrylic acid derivative (II) in the presence of a base, to produce a thiazoleglutaconic acid derivative (III) by means of a Michael-type addition:

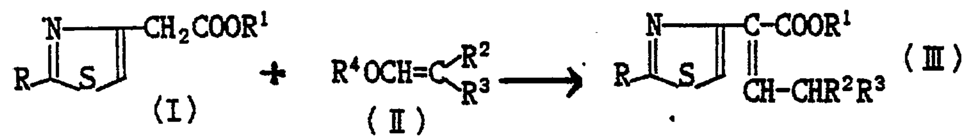
(I) (II) (III)

wherein:

R is an amino group which may be protected by ($C_1$–$C_5$)-alkanoyl, halogeno-($C_1$–$C_5$)-alkanoyl, ($C_2$–$C_6$)-alkoxycarbonyl, ($C_8$–$C_{15}$)-aralkoxycarbonyl, ($C_1$–$C_{15}$)-alkylsulfonyl, ($C_1$–$C_{15}$)-arylsulfonyl, ($C_{19}$–$C_{20}$)-triarylmethyl, a ($C_2$–$C_{10}$)-enamine-forming group, tri-($C_1$–$C_5$)-alkylsilyl, a ($C_2$–$C_{10}$)-Schiff base-forming group;

$R^1$ is an ester-forming group selected from ($C_1$–$C_8$)-alkyl; ($C_1$–$C_5$)-alkyl substituted by halogen or by ($C_1$–$C_5$)-alkoxy or by ($C_1$–$C_8$)-alkanesulfonyl; ($C_2$–$C_5$)-alkenyl; or ($C_7$–$C_{15}$)-aralkyl;

$R^2$ and $R^3$ each are hydrogen, cyano, or an esterified carboxyl group carrying one of the ester-forming groups defined for $R^1$; and

$R^4$ is ($C_1$–$C_5$)-alkyl or ($C_7$–$C_{15}$)-aralkyl.

2. A process as claimed in claim 1, which comprises the steps of:

– reacting the thiazoleacetic acid derivative (I) with the alkoxyacrylic acid derivative (II) in the presence of a base to produce a thiazolealkoxyglutaric acid derivative (Ib) by means of carbanion formation and addition,

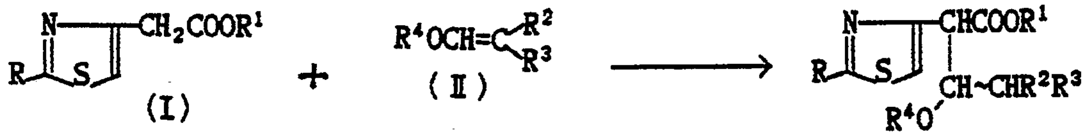
(I) (II)

– and reacting said thiazolealkoxyglutaric acid derivative (Ib) with a base to produce the desired thiazoleglutaconic acid derivative (III) by means of elimination of an alcohol $R^4OH$,

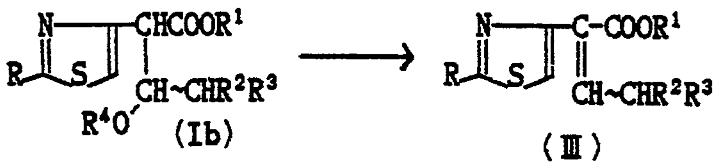
(Ib) (III)

wherein R, $R^1$, $R^2$, $R^3$ and $R^4$ have tha same meanings as in claim 1.

3. A process as claimed in claim 1, which comprises a reaction of the thiazoleacetic acid derivative (I) with the alkoxyacrylic acid derivative (II) and the base in one single step.

4. A process as claimed in claim 3, wherein $R^2$ is hydrogen.

5. A process as claimed in claims 1–4, wherein the base is an alkalimetal hydride or alkoxide.

6. A process as claimed in claims 1–5, wherein the steps are carried out in a solvent selected from hydrocarbons, ethers, nitriles, sulfoxides, amides, esters or mixtures thereof.

7. A process as claimed in claims 1–5, wherein the alkoxyacrylic acid derivative (II) is used as a reaction solvent in at least one of the process steps.

8. A process as claimed in claims 1–7, wherein 1 to 3 molar equivalents of the alkoxyacrylic acid derivative (II) are treated with 1 molar equivalent of the thiazoleacetic acid derivative (I) in the presence of 2 to 5 molar equivalents of a base and in 3 to 20 parts by weight of a solvent at a temperature between –20° and +70°C and during a period ranging from 5 minutes to 10 hours.

9. Process as claimed in claims 1–8, wherein the product is hydrolyzed (followed by decarboxylation when $R^2$ and $R^3$ are both carboxyl) to produce a thiazoleglutaconic acid derivative (IV):

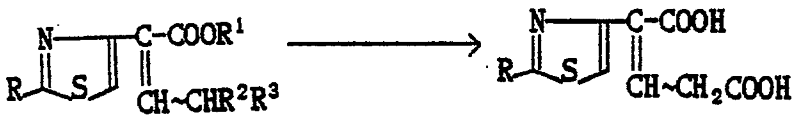

(III) (IV)

wherein R, $R^1$, $R^2$, $R^3$ have the same meanings as in claim 1.

## Patentansprüche

1. Verfahren zur Herstellung eines Thiazolglutaconsäurederivats, dadurch gekennzeichnet, daß ein Thiazolessigsäurederivat (I) mit einem Alkoxyacrylsäurederivat (II) in Anwesenheit einer Base unter Bildung eines Thiazolglutaconsäurederivats (III) mittels einer Michael-Typ-Addition umgesetzt wird:

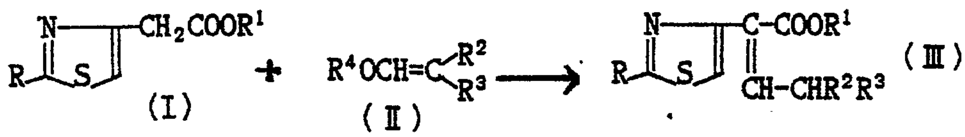

(I) (II) (III)

worin:

R eine Aminogruppe, die durch $(C_1–C_5)$-Alkanoyl, Halogeno-$(C_1–C_5)$-alkanoyl, $(C_2–C_6)$-Alkoxycarbonyl, $(C_8–C_{15})$-Aralkoxycarbonyl, $(C_{1-5})$-Alkylsulfonyl, $(C_1–C_{15})$-Arylsulfonyl, $(C_{19}–C_{20})$-Triarylmethyl, eine $(C_3–C_{10})$-Enamin-bildende Gruppe, Tri$(C_1–C_5)$-alkylsilyl, eine $(C_2–C_{10})$-Schiff'sche Base-bildende Gruppe geschützt sein kann, bedeutet;

$R^1$ eine Ester-bildende Gruppe, ausgewählt unter $(C_1–C_8)$-Alkyl, $(C_1–C_5)$-Alkyl, substituiert durch Halogen oder durch $(C_1–C_5)$-Alkoxy oder durch $(C_1–C_8)$-Alkansulfonyl; $(C_2–C_5)$-Alkenyl oder $(C_7–C_{15})$-Aralkyl, bedeutet;

$R^2$ und $R^3$ je Wasserstoff, Cyano oder eine veresterte Carboxylgruppe, die eine der Ester-bildenden Gruppen, wie sie für $R^1$ definiert wurden, enthält, bedeuten; und

$R^4$ $(C_1–C_5)$-Alkyl oder $(C_7–C_{15})$-Aralkyl bedeutet.

2. Verfahren nach Anspruch 1, gekennzeichnet durch die folgenden Stufen:

– Umsetzung des Thiazolessigsäurederivats (I) mit dem Alkoxyacrylsäurederivat (II) in Anwesenheit einer Base unter Bildung eines Thiazolalkoxyglutarsäurederivats (Ib) mittels Carbanionbildung und Addition:

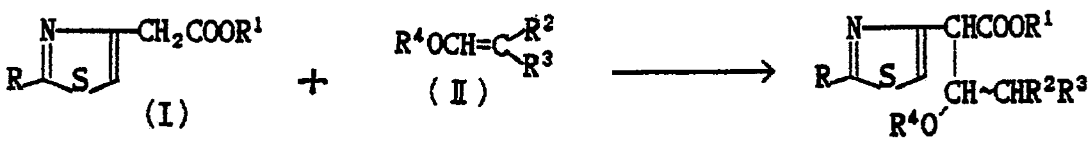

(I) (II)

– und Umsetzung des Thiazolalkoxyglutarsäurederivats (Ib) mit einer Base unter Bildung des gewünschten Thiazolglutaconsäurederivats (III) durch Eliminierung eines Alkohols $R^4OH$:

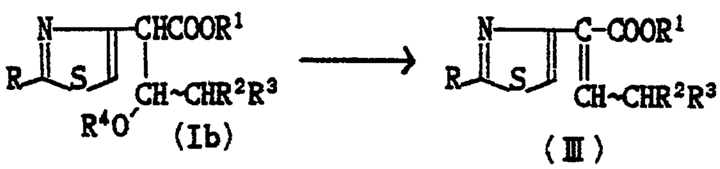

(Ib) (III)

worin R, $R^1$, $R^2$, $R^3$ und $R^4$ die in Anspruch 1 angegebenen Bedeutungen besitzen.

3. Verfahren nach Anspruch 1, dadurch gekennzeichnet, daß die Umsetzung des Thiazolessigsäurederivats (I) mit dem Alkoxyacrylsäurederivat (II) und der Base in einer einzigen Stufe durchgeführt wird.

4. Verfahren nach Anspruch 3, dadurch gekennzeichnet, daß $R^2$ Wasserstoff bedeutet.

5. Verfahren nach einem der Ansprüche 1 bis 4, dadurch gekennzeichnet, daß die Base ein Alkalimetallhydrid oder -alkoxid ist.

6. Verfahren nach einem der Ansprüche 1 bis 5, dadurch gekennzeichnet, daß die Stufen in einem Lösungsmittel, ausgewählt unter Kohlenwasserstoffen, Ethern, Nitrilen, Sulfoxiden, Amiden, Estern oder ihren Gemischen, durchgeführt werden.

7. Verfahren nach einem der Ansprüche 1 bis 5, dadurch gekennzeichnet, daß das Alkoxyacrylsäurederivat (II) als Reaktions-Lösungsmittel bei mindestens einer der Verfahrensstufen verwendet wird.

8. Verfahren nach einem der Ansprüche 1 bis 7, dadurch gekennzeichnet, daß 1 bis 3 Mol-Äquivalente Alkoxyacrylsäurederivat (II) mit 1 Mol-Äquivalent Thiazolessigsäurederivat (I) in Anwesenheit von 2 bis 5 Mol-Äquivalenten einer Base und 3 bis 20 Gew.-Teilen eines Lösungsmittels bei einer Temperatur zwischen −20 und +70°C und während einer Zeit im Bereich von 5 Minuten bis 10 Stunden behandelt werden.

9. Verfahren nach einem der Ansprüche 1 bis 8, dadurch gekennzeichnet, daß das Produkt hydrolysiert wird (gefolgt von einer Decarboxylierung, wenn $R^2$ und $R^3$ beide Carboxyl bedeuten), wobei ein Thiazolglutaconsäurederivat (IV):

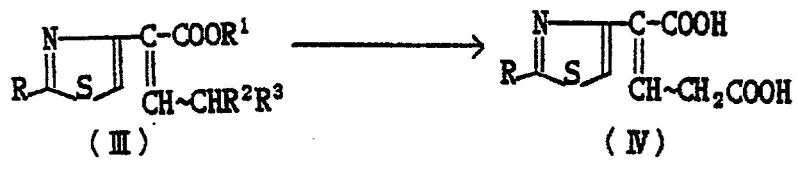

gebildet wird, worin R, $R^1$, $R^2$, $R^3$ die in Anspruch 1 gegebenen Bedeutungen besitzen.

**Revendications**

1. Procédé de préparation d'un dérivé d'acide thiazoleglutaconique, qui comprend la réaction d'un dérivé d'acide thiazolacétique (I) avec un dérivé d'acide alcoxyacrylique (II) en présence d'une base, pour produire un dérivé d'acide thiazoleglutaconique (III) par une addition de type Michael:

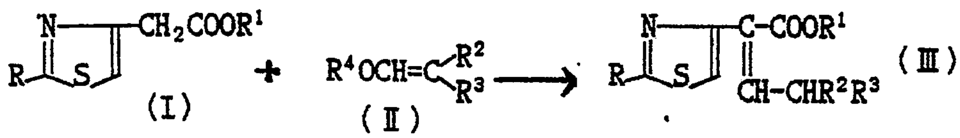

où:

R est un groupe amino qui peut être protégé par alcanoyle en $C_1$–$C_5$, halogénoalcanoyle en $C_1$–$C_5$, alcoxycarbonyle en $C_2$–$C_6$, aralcoxycarbonyle en $C_8$–$C_{15}$, alkylsulfonyle en $C_1$–$C_{15}$, arylsulfonyle en $C_1$–$C_{15}$, triarylméthyle en $C_{19}$–$C_{20}$, un groupe de formation d'une énamine, en $C_3$–$C_{10}$, tri(alkyl en $C_1$–$C_5$)silyle, un groupe de formation d'une base de Schiff, en $C_2$–$C_{10}$;

$R^1$ représente un groupe de formation d'ester choisi parmi alkyle en $C_1$–$C_8$; alkyle en $C_1$–$C_5$ substitué par halogène ou par alcoxy en $C_1$–$C_5$ ou par alcanesulfonyle en $C_1$–$C_8$; alcényle en $C_2$–$C_5$; ou aralkyle en $C_7$–$C_{15}$;

$R^2$ et $R^3$ représentent chacun hydrogène, cyano, ou un groupe carboxyle estérifié portant l'un des groupes de formation d'ester définis pour $R^1$; et

$R^4$ représente alkyle en $C_1$–$C_5$ ou aralkyle en $C_7$–$C_{15}$.

2. Procédé selon la revendication 1, qui comprend les étapes consistant à:

– faire réagir le dérivé d'acide thiazoleacétique (I) avec le dérivé d'acide alcoxyacrylique (II), en présence d'une base, pour obtenir un dérivé d'acide thiazolealcoxyglutarique (Ib), par formation de carbanion et addition,

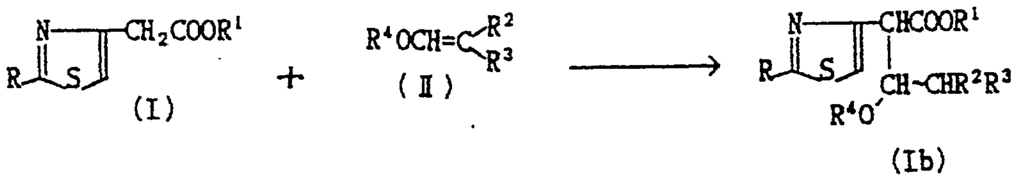

; et

– faire réagir ledit dérivé d'acide thiazolealcoxyglutarique (Ib) avec une base, pour obtenir le dérivé d'acide thiazoleglutaconique (III) désiré, par élimination d'un alcool $R^4OH$,

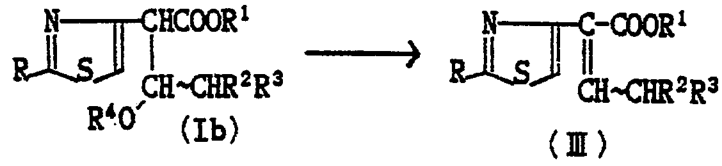

où R, $R^1$, $R^2$, $R^3$, et $R^4$ ont les mêmes significations qu'à la revendication 1.

3. Procédé selon la revendication 1, qui comprend une réaction du dérivé d'acide thiazoleacétique (I) avec le dérivé d'acide alcoxyacrylique (II) et la base, en une seule étape.

4. Procédé selon la revendication 3, dans lequel $R^2$ représente hydrogène.

5. Procédé selon l'une des revendications 1 à 4, dans lequel la base est un hydrure ou un alcoolate de métal alcalin.

6. Procédé selon l'une des revendications 1 à 5, dans lequel les étapes sont effectuées dans un solvant choisi parmi les hydrocarbures, les éthers, les nitriles, les sulfoxydes, les amides, les esters ou leurs mélanges.

7. Procédé selon l'une des revendications 1 à 5, dans lequel le dérivé d'acide alcoxyacrylique (II) est utilisé en tant que solvant de réaction dans au moins l'une des étapes du procédé.

8. Procédé selon l'une des revendications 1 à 7, dans lequel 1 à 3 équivalents molaires du dérivé d'acide alcoxyacrylique (II) sont traités par 1 équivalent molaire du dérivé d'acide thiazoleacétique (I), en présence de 2 à 5 équivalents molaires d'une base, et dans 3 à 20 parties en poids d'un solvant, à une température comprise entre –20°C et +70°C, et pendant une période de temps allant de 5 minutes à 10 heures.

9. Procédé selon l'une des revendications 1 à 8, dans lequel le produit est soumis à une hydrolyse (suivie d'une décarboxylation lorsque $R^2$ et $R^3$ représentent tous deux carboxyle), pour produire un dérivé d'acide thiazoleglutaconique (IV):

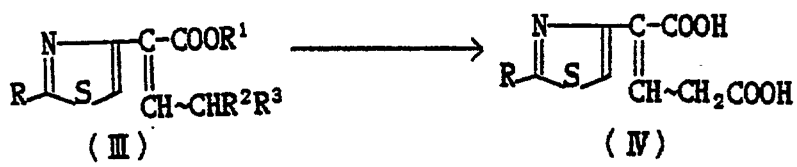

où R, $R^1$, $R^2$, $R^3$ ont les mêmes significations qu'à la revendication 1.